Europäisches Patentamt

European Patent Office

Office européen des brevets

(11) Veröffentlichungsnummer: **0 021 415**
A1

(12) **EUROPÄISCHE PATENTANMELDUNG**

(21) Anmeldenummer: **80103587.4**

(22) Anmeldetag: **25.06.80**

(51) Int. Cl.³: **A 61 N 5/02, A 61 B 6/00**

(30) Priorität: **25.06.79 DE 2925601**

(43) Veröffentlichungstag der Anmeldung: **07.01.81**
**Patentblatt 81/1**

(84) Benannte Vertragsstaaten: **FR GB IT**

(71) Anmelder: **Brück, Gernot Klaus,**
**Hermann-Pflaume-Strasse 6, D-5000 Köln 41 (DE)**

(72) Erfinder: **Brück, Gernot Klaus,**
**Hermann-Pflaume-Strasse 6, D-5000 Köln 41 (DE)**

(74) Vertreter: **Patentanwälte Dr. Solf & Zapf,**
**Postfach 13 02 19, D-5600 Wuppertal 1 (DE)**

(54) **Vorrichtung zur Strahlenbehandlung an lebenden Körpern.**

(57) Es handelt sich um eine Vorrichtung zur Behandlung von örtlich begrenzten Stellen in einem lebenden Körper mit einer eine Energiestrahlung aussendenden Strahlungsquelle (10) und einem den Strahlengang umgebenden, mindestens einen Teil eines Ellipsoidspiegels (2) bildenden Gehäuses, in dessen erstem Brennpunkt die Austrittsöffnung (11) der Strahlenquelle angeordnet ist und dessen zweiter Brennpunkt (6) den Bestrahlungsort bildet. Hierbei ist die abgestrahlte Strahlungsenergie der Strahlungsquelle (10) derart bemessen, daß die Energieeinstrahlung der Wärmeableitfähigkeit von gesundem Gewebe des lebenden Körpers derart angepaßt ist, daß bei einer bestimmten gewählten Höchsttemperatur im gesunden Gewebe die pro Zeiteinheit eingestrahlte Energie die von dem gesunden Gewebe abgeleitete Energie kompensiert.

- 1 -

## Vorrichtung zur Strahlenbehandlung an lebenden Körpern

Die vorliegende Erfindung betrifft eine Vorrichtung zur Behandlung von örtlich begrenzten Stellen in einem lebenden Körper mit einer eine Energiestrahlung aussendenden Strahlungsquelle und einem den Strahlengang umgebenden, mindestens einen Teil eines Ellypsoidspiegels bildenden Gehäuses, in dessen erstem Brennpunkt die Austrittsöffnung der Strahlenquelle angeordnet ist und dessen zweiter Brennpunkt den Bestrahlungsort bildet.

Eine derartige Vorrichtung ist bereits aus der deutschen Patentanmeldung P 2 452 227.8 bekannt. Diese bekannte Vorrichtung weist insoweit einen Nachteil auf, und ist deshalb verbesserungswürdig, weil nicht hinreichend sichergestellt ist, daß das gesunde Gewebe, das das zu bestrahlende Gewebe umgibt, möglichst keinen Schaden erleidet.

Demgemäß liegt der vorliegenden Erfindung die Aufgabe zugrunde, die vorstehend beschriebene Vorrichtung derart zu verbessern, daß das zu untersuchende Objekt nur an den tatsächlich zu bestrahlenden Stellen durch die Einwirkung der Energiestrahlung derart erwärmt wird, daß die zu bestrahlende Objektstelle, d. h. das jeweilig erkrankte Gewebe, einer bleibenden Temperaturbeeinflussung unterworfen und exakt getroffen wird.

0021415

Erfindungsgemäß wird dies durch eine Strahlungsquelle erreicht, deren abgestrahlte Strahlungsenergie derart bemessen ist, daß die Energieeinstrahlung der Wärmeableitfähigkeit des gesunden Gewebes derart angepaßt ist, daß bei einer bestimmten gewählten Höchsttemperatur im gesunden Gewebe die pro Zeiteinheit eingestrahlte Energie die von dem gesunden Gewebe abgeleitete Energie kompensiert. Die Erfindung basiert dabei auf der Erkenntnis, daß krankes, insbesondere tumorartiges Gewebe eine geringere Wärmeableitfähigkeit besitzt als gesundes Gewebe, d.h. durch die erfindungsgemäß gesteuerte Strahlungsquelle wird sich das gesunde Gewebe nach der Erwärmung durch die eingestrahlte Energie nur auf eine bestimmte Höchsttemperatur erwärmen und die Temperatur dann konstant beibehalten, während das kranke Gewebe, das eine geringere Wärmeableitfähigkeit besitzt, sich in derselben Zeit ständig weiter erwärmt. Auf diese Weise kann also innerhalb des kranken Gewebes eine Temperaturerhöhung erreicht werden, und zwar derart, daß das kranke Gewebe zerstört wird, während das gesunde Gewebe nicht unzulässig temperaturbelastet wird. In weiterer Ausgestaltung der Erfindung kann eine Strahlungsquelle mit pulsierender Abstrahlung einzelner, in bestimmten Zeitintervallen aufeinanderfolgender Strahlungsimpulse verwendet werden, wobei die Länge der Zeitintervalle der Abkühlungszeit vom gesunden Gewebe nach erfolgter Erwärmung durch den vorangehenden Impuls entspricht.

Dabei wird durch die erfindungsgemäß gesteuerte Strahlungsquelle sich das gesunde Gewebe nach der Erwärmung durch den jeweiligen Energieimpuls in der Impulsintervallzeit wieder auf die Ursprungstemperatur abkühlen, während das kranke Gewebe, das eine geringere Wärmeab-

0021415

leitfähigkeit besitzt, sich in derselben Impulsinterwallzeit nicht vollständig auf die Ursprungstemperatur
vor dem jeweiligen Energieimpuls abkühlen kann, so daß
das kranke, insbesondere tumorartige Gewebe nach jedem
Energieimpuls eine höhere Temperatur aufweist als vor
dem jeweiligen Energieimpuls. Die Strahlungsimpulse
besitzen einen Impulsabstand, dessen Länge der Abkühlungszeit vom gesunden Gewebe nach erfolgter Erwärmung
durch den jeweils vorangehenden Impuls entspricht.
Hierbei ist es erfindungsgemäß wichtig, wenn die Impulshöhe der einzelnen Strahlungsimpulse derart bemessen ist,
daß die dadurch in dem zu bestrahlenden Objekt erzeugte
Temperaturerhöhung nicht die Objektstruktur, insbesondere das Gewebe des zu bestrahlenden Körpers schädigt.
Vielmehr wird erfindungsgemäß erreicht, daß durch die
Summe der aufeinanderfolgenden Impulse in der kranken
Gewebestruktur lediglich eine derartige Temperaturerhöhung erzielt wird, daß die temperaturanfälligen bzw.
temperaturempfindlichen Gewebezellen unter der erhöhten
Temperatur absterben. Dabei bleibt das gesunde Gewebe
ungeschädigt, da dieses sich jeweils nach jedem Impuls
wieder auf die Ursprungstemperatur abkühlen kann. Dabei
kann es erfindungsgemäß weiterhin vorteilhaft sein,
wenn die Impulshöhe der aufeinanderfolgenden Strahlungsimpulse über die Bestrahlungszeit abnimmt. Dies kann
deshalb erforderlich bzw. günstig sein, da lediglich
eine bestimmte Temperaturerhöhung im kranken Gewebe
erforderlich ist, z. B. bei dem menschlichen Körper
eine Temperaturerhöhung von ca. 36,8° C auf 44 bis
45° C, so daß, wenn die angestrebte Temperaturerhöhung erreicht ist, jeweils nur noch Strahlungsimpulse
von geringer Höhe ausreichend sind, um die Endtempera-

0021415

tur zu halten. Daneben kann aber auch noch zusätzlich der Impulsintervallabstand bzw. die Intervallzeit der einzelnen Impulse variiert werden, und zwar in bezug auf eine Intervallverlängerung, um auf diese Weise, d. h. durch Kombination der Impulshöhenverringerung und der Impulsabstandszeit, eine konstante Endtemperatur im kranken Gewebe über längere Zeit aufrechtzuerhalten. Vorteilhafterweise liegt die Wellenlänge der Impulsstrahlung im Bereich von 0,1 bis 100 mm, insbesondere zwischen 10 und 100 mm.

In weiterer Ausgestaltung der erfindungsgemäßen Vorrichtung kann es zweckmäßig sein, wenn in der horizontalen Ebene oder unmittelbar unter der horizontalen Ebene des zweiten Brennpunktes ein Auflager für das zu bestrahlende Objekt angeordnet ist, das aus einem in seinem Füllungsgrad regulierbaren Polsterkissen besteht. Durch diese erfindungsgemäße Maßnahme kann der Bestrahlungsort genau einjustiert werden, indem das als Auflager dienende Kissen mehr oder weniger aufgefüllt wird, wodurch der innerhalb des zu bestrahlenden Körpers fallende zweite Brennpunkt innerhalb des Körpers in vertikaler Ebene, d. h. in Richtung der Längsachse des Ellipsoidspiegels verschoben wird. Da

- 5 -

0021415

sich der Füllungsgrad eines derartigen Kissens stufenlos ändern läßt, ist somit eine sehr feine Verstellung und Einjustierung möglich. Erfindungsgemäß ist es dabei weiterhin von Vorteil, wenn das Kissen mit einem Medium gefüllt ist, dessen Dielektrizitätskonstante derjenigen des zu bestrahlenden Objektes entspricht oder weitgehenst angenähert ist.

Um die Strahlungsintensität noch zu verbessern, kann es erfindungsgemäß vorteilhaft sein, wenn der Ellipsoidspiegel als Vollellipse ausgeführt ist, wodurch ein Strahlungsverlust weitgehend ausgeschaltet wird und auch die von der Unterseite des zu bestrahlenden Objektes anfallende Strahlung voll ausgewertet werden kann. Dabei ist vorgesehen, daß in den Seitenwänden des Ellipsoidspiegels in der horizontalen Ebene des zweiten Brennpunktes jeweils Öffnungen vorgesehen sind, die einander gegenüberliegen, so daß beispielsweise wenn ein Mensch im Bereich der Brust oder des Unterleibs bestrahlt werden soll, seine Gliedmaße und der Kopf jeweils aus dem Ellipsoidspiegel herausragen, und zwar durch die Öffnungen hindurch.

Anhand der in den beiliegenden Figuren dargestellten Ausführungsbeispiele wird die Erfindung nun näher erläutert. Es zeigen:

Fig. 1 einen Schnitt durch eine erfindungsgemäße Vorrichtung in einer ersten Ausführungsform,

Fig. 2 einen Schnitt durch eine erfindungsgemäße Vorrichtung unter Verwendung eines als geschlossenes Ellipsoid ausgebildeten Ellipsoidspiegels,

Fig. 3 eine Darstellung der Impulsfolge der erfindungsgemäß verwendeten Strahlungsquelle und der einerseits im gesunden Gewebe und andererseits im kranken Gewebe erfolgten Temperaturerhöhung über die Zeit.

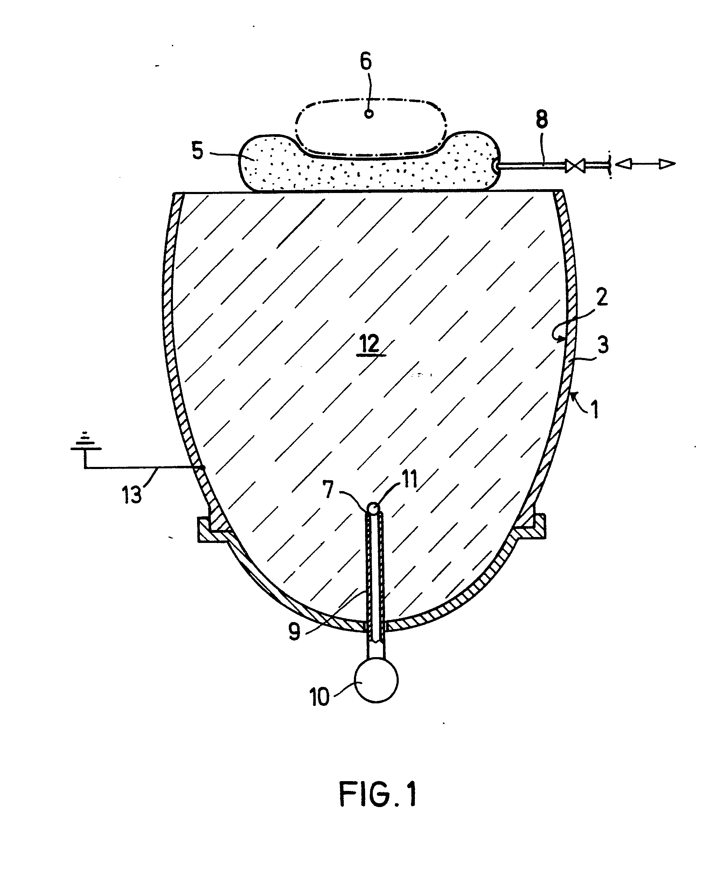

Wie aus Fig. 1 zu ersehen ist, besteht eine erfindungsgemäße Vorrichtung aus einem Gehäuse 1, das in seinem inneren Teil einen Teil, d. h. im dargestellten Beispiel etwa mehr als zwei Drittel, eines Ellipsoidspiegels 2 bildet. Das Gehäuse 1 umfaßt einen breiteren Gehäusering 3 und eine auf diesen aufegesetzte Gehäusekappe 4, auf deren innerer Oberfläche eine Endkalotte des Ellipsoidspiegels 2 ausgebildet ist.

Auf der von der Gehäusekappe 4 abgewendeten Seite können, wie in der bekannten Patenanmeldung P 2 452 227.8 beschrieben ist, sich eine Vielzahl von kleinen Gehäuseringen anschließen, die in lösbarer Weise dicht aneinandergesetzt sind. Dabei sind die aneianderstoßenden Kanten der einzelnen Gehäuseteile so gearbeitet, daß im zusammengesetzten Zustand der Teile der im Inneren des Gehäuses gebildete Ellipsoidspiegel so wenig wie möglich unterbrochen ist. Im vorliegenden Beispiel ist jedoch der Gehäusering 3 einstückig ausgebildet, da die Einjustierung des Brennpunktes im zu bestrahlenden Objekt mittels eines als Auflager für dieses dienende Kissen 5 erfolgt. Dieses Kissen 5 ist derart angeordnet, daß seine Oberfläche vorteilhafterweise unterhalb des zweiten Brennpunktes des Ellipsoidspiegels 2 liegt. Der zweite Brennpunkt ist mit der Ziffer 6 gekennzeichnet. Der erste Brennpunkt des Ellipsoidspiegels 2 liegt im Inneren des Gehäuses 1 und ist mit 7 gekennzeichnet. Bei dem Kissen 5 handelt es sich um ein mit einem Medium über eine Druckleitung 8 füllbares oder entleerbares Kissen. Somit kann durch die Veränderung des Füllungsgrads des Kissens 5 die Lage des zu bestrahlenden Objekts in bezug auf den zweiten Brennpunkt in Richtung der großen Ellipsenachse verändert werden, wodurch eine genaue Einstellung des zweiten Brennpunktes auf die jeweils zu bestrahlende, z. B. tumorartiges Gewebe aufweisende Objektstelle erfolgen kann.

Ein Hohlleiter 9 ist durch eine Öffnung im Scheitel der Gehäusekappe 4 bis zu dem ersten Brennpunkt 7 geführt. An dem außerhalb des Gehäuses 1 liegenden Ende des Hohlleiters 9 ist eine Strahlungsquelle 10 angeordnet, während das im ersten Brennpunkt 7 des Ellipsoidspiegels 2 liegende Ende des Hohlleiters 9 eine Austrittsöffnung 11 für die Strahlung bildet.

Sowohl der Hohlleiter 9 als auch das gesamte Gehäuse 1 sind zweckmäßigerweise mit einem Dielektrikum 12 ausgefüllt, dessen Dielektrizitätskonstante der Dielektrizitätskonstanten eines lebenden Körpers, der bestrahlt werden soll, so weit wie möglich angenähert ist. Diese Dielektrizitätskonstante beträgt bei dem menschlichen Körper etwa .......

Der Ellipsoidspiegel 2 ist elektrisch leitfähig ausgebildet und zweckmäßigerweise mit einer Erdungsleitung 13 elektrisch verbunden.

Das in Fig. 1 gezeigte Kissen besitzt zweckmäßigerweise eine Hubhöhe, die mindestens der halben Höhe des zu bestrahlenden Objekts entspricht. Dadurch ist sichergestellt, daß das Objekt über die gesamte Körperdicke bzw. Höhe bestrahlt werden kann, wobei lediglich, wenn tiefer gelegene Bereiche innerhalb des Körpers bestrahlt werden sollen, eine Wendung des zu bestrahlenden Objekts um 180° zu erfolgen hat.

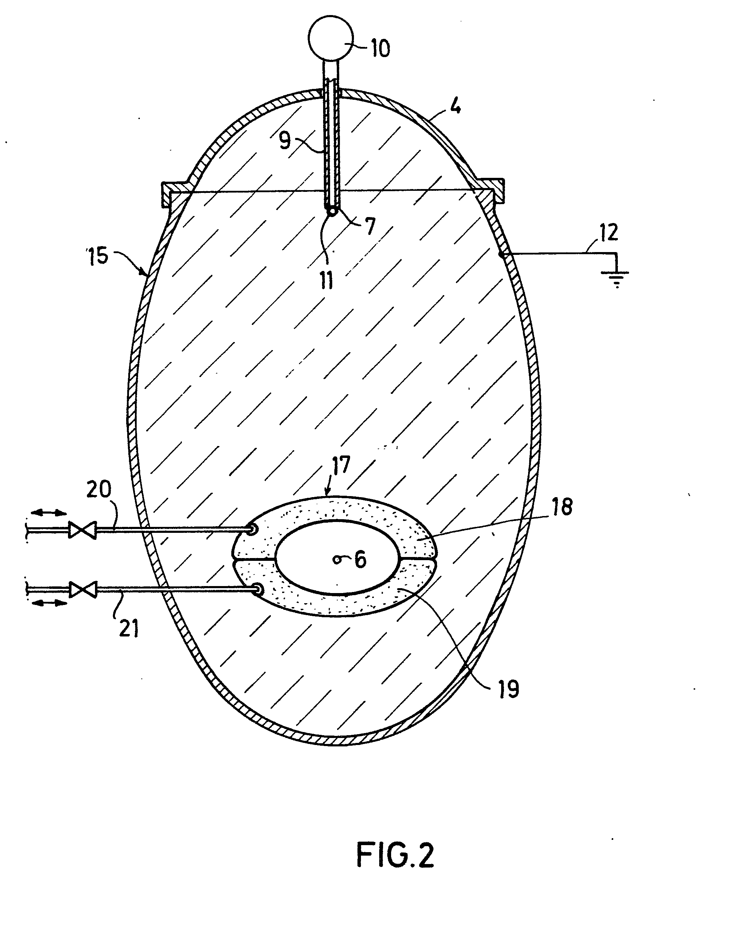

In Fig. 2 ist eine weitere Ausführungsform einer erfindungsgemäßen Vorrichtung gezeigt. Hierbei ist das Gehäuse 15 als geschlossene Ellipse ausgebildet, so daß der im Inneren sich erstreckende Ellipsoidspiegel 16 im gezeigten Querschnitt in Richtung der größeren Ellipsenachse eine ge-

0021415

schlossene Ellypsenform besitzt. Die Ausführungsform hat den Vorteil, daß keinerlei Strahlung praktisch verlorengeht, die aus der Austrittsöffnung 11 der Strahlenquelle 10 austritt. Im übrigen sind gleiche Teile der erfinungsgemäßen Vorrichtung wie bei der Fig. 1 mit denselben Bezugsziffern versehen. Als Auflager für das im zweiten Brennpunkt 6 anzuordnende Bestrahlungsobjekt dient bei dem dargestellten Beispiel ein Kissen 17. Dieses Kissen 17 ist mit einem Druckmedium füllbar, dessen Dielektrizitätskonstante zweckmäßigerweise derjenigen des zu bestrahlenden Körpers weitgehend angepaßt ist. Das Kissen 17 ist aus zwei voneinander getrennten Teilen, dem Unterteil 18 und dem Oberteil 19, gebildet. Beide Kissenteile 18, 19 sind durch separate Druckleitungen füllbar oder entleerbar. Diese Druckleitungen sind mit 20, 21 gekennzeichnet. Diese Ausführungsform des Kissens 17 besitzt den Vorteil, daß der zu bestrahlende Körper von beiden Seiten eingeschlossen ist und somit der Ellipsoidspiegel mit seinem mit Dielektrikum gefüllten Raum, dessen Dielektrizitätskonstante ebenfalls weitgehend derjenigen des zu bestrahlenden Körpers angepaßt ist, an das Kissen 17 angrenzt, so daß geringste Übergansverluste auftreten. Indem nun beispielsweise im unteren Kissenteil der Druck erhöht wird und gleichzeitig entsprechend im oberen Kissenteil der Druck erniedrigt wird, kann der Körper in Richtung auf den ersten Brennpunkt angehoben werden, wodurch eine Verschiebung des zweiten Brennpunktes nach unten im zu bestrahlenden Körper erfolgt. Umgekehrt erfolgt eine Verschiebung des zweiten Brennpunktes im zu bestrahlenden Körper nach oben, wenn im Kissenoberteil der Druck erhöht und gleichzeitig im Kissenunterteil der Druck erniedrigt wird. Auch hierbei ist der Kissenhub der beiden Kissenteile derart bemessen, daß er mindestens der halben Körperdicke des zu bestrahlenden Körpers entspricht. Diese Ausführungsform des Kissens besitzt den Vorteil, daß der Ellipsoidspiegel inklusive dem Gehäuse selbst nicht justiert werden muß, vielmehr starr angeordnet sein kann. Zweckmäßigerweise ist das Gehäuse 15 derart bemessen, daß beispielsweise ein Mensch mit einem bestimmten Körperabschnitt, der zu bestrahlen ist, innerhalb des Gehäuses liegend angeordnet werden kann, wobei dann die Beine und der Kopf bzw. der Oberkörper aus dem Gehäuse 1 an gegenüberliegenden Gehäuseöffnungen, die in der Ebene des zweiten Brennpunktes angeordnet sind, hinausragen.

Die in Fig. 2 gezeigte Ausführungsform des Kissens 17 kann auch bei der Ausführungsform gem. Fig. 1 verwendet werden, wobei dann dort die gleichen Vorteile wie bei der Ausführungsform gem. Fig. 2 erzielt werden, nämlich daß eine Justierung des Gehäuses 1 selbst nicht mehr erfolgen muß.

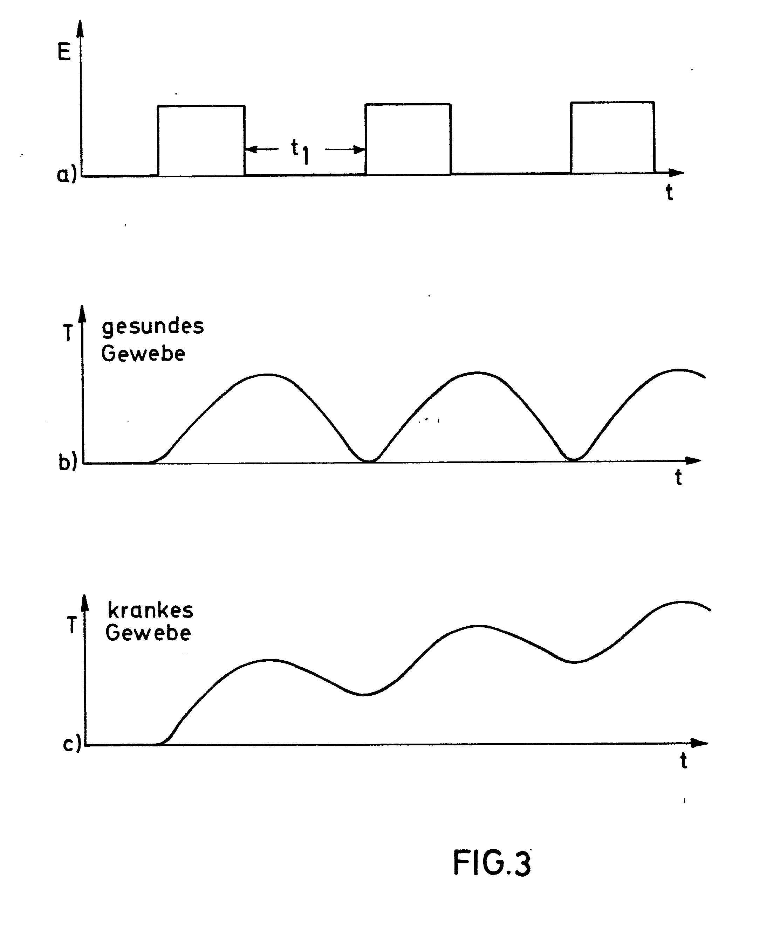

In Fig. 3 ist anhand der zeitlichen Impulsfolge der Strahlungsquelle gezeigt, wie die Wirkungsweise der erfindungsgemäßen Vorrichtung sich vollzieht. Die Strahlungsquelle sendet in einer bestimmten Impulsfolge auftretende Rechteckimpulse einer Energiestrahlung mit einer Wellenlänge im Bereich von vorzugsweise 10 bis 100 mm. Dabei ist die Impulshöhe derart gewählt, daß die dadurch verursachte Erwärmung des Körpergewebes, und zwar des gesunden Körpergewebes, wie in Fig. 3b dargestellt, nicht einen die Gewebestruktur zerstörenden Wert erreicht. Ein derartiger Wert sollte etwa zwischen 37 und 38° Celsius liegen, wenn von einer durchschnittlichen Körpertemperatur beim Menschen von 36,8° Celsius ausgegangen wird. Indem nun die Impulsfolge 2 zwischen zwei aufeinanderfolgenden Impulsen derart gewählt ist, diese ist mit t1 gekennzeichnet, daß sie gleich oder größer der Abklingzeit der Erwärmung im gesunden Gewebe auf die Ausgangstemperatur ist, wird erreicht, daß innerhalb dieser Intervallzeit t1, wie in Fig. 3b gezeigt, die Temperatur im gesunden Gewebe sich wieder auf die Ausgangstemperatur verringert. Aufgrund der geringeren Wärmeableitfähigkeit von krankem, tumorartigem Gewebe wird aber erreicht, daß in derselben Zeit sich die Temperatur im kranken Gewebe nicht auf den Ausgangswert zu Beginn der Bestrahlung verringert, sondern nach dem Ende der Intervallzeit noch eine erhöhte Temperatur innerhalb des kranken Gewebes herrscht. Durch den folgenden Impuls erfolgt nun wieder eine Erwärmung sowohl des gesunden Gewebes als auch des kranken Gewebes, jedoch jeweils von unterschiedlichen Ausgangstemperaturen, was bedeutet, daß sich im kranken Gewebe die Temperatur weiter erhöht, während im gesunden Gewebe lediglich wiederum nur eine bestimmte Temperatur erreicht wird, die auch beim vorhergehenden Impuls erreicht worden ist.Durch eine längere Impulsfolge wird demnach eine laufende Erhöhung der Temperatur im kranken Gewebe erzielt, während im gesunden Gewebe lediglich eine beständige Temperaturschwankung zwischen zwei unbedenklichen und nicht schädlichen Temperaturwerten erfolgt. Dabei kann die Erhöhung im kranken Gewebe auf eine Temperatur durchgeführt werden, bei der das kranke Gewebe abstirbt. Eine derartige Temperatur liegt bei menschlichem Körpergewebe im Bereich von 44 bis 45° Celsius. Gleichzeitig aber wird dabei das gesunde Gewebe geschont, so daß hieran keine Schädigungen zu befürchten sind. Indem nun die Impulsfolge der einzelnen Impulse mit zunehmender Temperatur im kranken Gewebe verlängert wird, kann schließlich erreicht werden, daß die Temperatur im kranken Gewebe auf einem konstanten Wert gehalten werden kann, wozu es weiterhin zweckmäßig ist, auch gleichzeitig die Impulshöhe zu verringern.

- 1 -

<u>Ansprüche</u>

1. Vorrichtung zur Behandlung von örtlich begrenzten Stellen in einem lebenden Körper mit einer eine Energiestrahlung aussendenden Strahlungsquelle und einem den Strahlengang umgebenden, mindestens einen Teil eines Ellipsoidspiegels bildenden Gehäuses, in dessen erstem Brennpunkt die Austrittsöffnung der Strahlenquelle angeordnet ist und dessen zweiter Brennpunkt den Bestrahlungsort bildet, g e k e n n z e i c h n e t   d u r c h eine Strahlungsquelle (10),deren abgestrahlte Strahlungsenergie derart bemessen ist, daß die Energieeinstrahlung der Wärmeableitfähigkeit des gesunden Gewebes derart angepaßt ist, daß bei einer bestimmten gewählten Höchsttemperatur im gesunden Gewebe die pro Zeiteinheit eingestrahlte Energie die von dem gesunden Gewebe abgeleitete Energie kompensiert.

2. Vorrichtung nach Anspruch 1, d a d u r c h   g e - k e n n z e i c h n e t, daß die Strahlungsquelle (10) eine pulsierende Abstrahlung einzelner, in bestimmten Zeitintervallen (t1) aufeinanderfolgender Strahlungsimpulse aufweist.

3. Vorrichtung nach Anspruch 2, d a d u r c h g e - k e n n z e i c h n e t , daß die Strahlungsimpulse einen Impulsabstand (t1) aufweisen, dessen Länge der Abkühlungszeit von gesundem Gewebe nach erfolgter Erwärmung entspricht oder größer als diese ist.

4. Vorrichtung nach Anspruch 2 oder 3, d a d u r c h g e k e n n z e i c h n e t , daß die Impulshöhe der Strahlungsimpulse derart bemessen ist, daß die dadurch in dem zu bestrahlenden Körper erzeugte Temperaturerhöhung nicht die Körperstruktur, insbesondere das Körpergewebe, schädigt.

5. Vorrichtung nach einem oder mehreren der Ansprüche 2 bis 4, d a d u r c h g e k e n n z e i c h n e t , daß die Impulshöhe der aufeinanderfolgenden Strahlungsimpulse über die Bestrahlungszeit abnimmt und/ oder der Impulsabstand zunimmt.

6. Vorrichtung nach einem oder mehreren der Ansprüche 1 bis 5, d a d u r c h g e k e n n z e i c h n e t , daß die Wellenlänge der Strahlung im Bereich von 0,1 bis 100 mm, insbesondere zwischen 10 und 100 mm,liegt.

7. Vorrichtung nach einem oder mehreren der Ansprüche 1 bis 6, d a d u r c h g e k e n n z e i c h n e t , daß das den Ellipsoidspiegel (16) bildende Gehäuse (15) im Querschnitt eine geschlossene Ellipse bildet mit in der Ebene des Bestrahlungsortes einander gegenüberliegenden Öffnungen in der Gehäusewandung zum Durchlaß nicht zu bestrahlender Körperteile.

8. Vorrichtung nach einem oder mehreren der Ansprüche 1 bis 7, d a d u r c h   g e k e n n z e i c h n e t, daß in oder unterhalb der horizontalen Ebene des zweiten Brennpunktes (6) ein Auflager für das zu bestrahlende Objekt angeordnet ist, das aus einem in seinem Füllungsgrad regulierbaren Kissen (5, 17) besteht.

9. Vorrichtung nach Anspruch 8, d a d u r c h   g e k e n n z e i c h n e t, daß das Kissen (17) aus zwei unabhängig voneinander in ihrem Füllungsgrad regelbaren Teilen (18, 19) besteht, die das zu bestrahlende Objekt zweiseitig umschließen.

10. Vorrichtung nach Anspruch 8 oder 9, d a d u r c h   g e k e n n z e i c h n e t, daß das Kissen (5, 17) mit einem Dielektrium gefüllt ist, dessen Dielektrizitätskonstante derjenigen des zu bestrahlenden Objektes entspricht bzw. weitgehend angenähert ist.

11. Vorrichtung nach einem oder mehreren der Ansprüche 1 bis 10, d a d u r c h   g e k e n n z e i c h n e t, daß die Spiegelfläche des Ellipsoidspiegels (2, 16) leitfähig ausgebildet und mit einer Erdnungsleitung (12) elektrisch verbunden ist.

12. Vorrichtung nach einem oder mehreren der Ansprüche 1 bis 11, d a d u r c h   g e k e n n z e i c h n e t, daß zwischen der Strahlungsquelle (10) und dem ersten Brennpunkt (7) ein Hohlleiter (9) angeordnet ist.

0021415

13. Vorrichtung nach einem oder mehreren der Ansprüche 1 bis 12, d a d u r c h   g e k e n n z e i c h - n e t, daß der Hohlleiter (9) und der Innenraum des Gehäuses (1,15) mit einem Dielektrikum ausgefüllt sind, dessen Dielektrizitätskonstante gleich oder annähernd gleich der Dielektrizitätskonstanten des zu bestrahlenden Körpers ist.

FIG.1

**FIG.2**

FIG.3

0021415

12

# EUROPÄISCHER RECHERCHENBERICHT

**Europäisches Patentamt**

Nummer der Anmeldung
EP 80103587.4

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>DE - A1 - 2 736 380</u> (BOSCH)<br>+ Seite 1, Patentansprüche 1-3; Seite 8, Zeile 16; Seite 10, Absatz 2 +<br>-- | 1,8,10 |
| | <u>DE - A1 - 2 508 494</u> (SCHULZ)<br>+ Seite 4, Zeilen 23-26,30,31; Seite 9, Zeilen 4-15; Seite 12, Patentansprüche 14-16 +<br>-- | 1-5,12, 13 |
| | <u>DE - A1 - 2 453 067</u> (REDDIG)<br>+ Seite 2, Patentanspruch 1 +<br>-- | 8-10 |
| D,X | <u>DE - A1 - 2 452 227</u> (BRÜCK)<br>+ Seite 9, Patentansprüche 2,3; Seite 10, Patentansprüche 7,8; Seite 11, Patentansprüche 9-11 +<br>-- | 6,11-13 |
| X | <u>DE - A1 - 2 417 263</u> (SCHULZ)<br>+ Seite 16, Patentansprüche 7-9; Seiten 18,19, Patentanspruch 24 +<br>-- | 6,12,13 |
| | <u>FR - A1 - 2 301 965</u> (DIAPULSE CORP.)<br>+ Seite 1, Zeile 1 - Seite 2, Zeile 6; Seiten 13,14, Patentanspruch 1 +<br>---- | 1-5 |

**KLASSIFIKATION DER ANMELDUNG (Int.Cl. 3)**

A 61 N 5/02
A 61 B 6/00

**RECHERCHIERTE SACHGEBIETE (Int. Cl. 3)**

A 61 N
A 61 B

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |

| Recherchenort | Abschlußdatum der Recherche | Prüfer |
|---|---|---|
| WIEN | 26-09-1980 | NEGWER |

EPA form 1503.1   06.78